# EUROPÄISCHE PATENTSCHRIFT

(11) **EP 1 051 132 B1**
(45) Veröffentlichungstag und Bekanntmachung des Hinweises auf die Patenterteilung: **20.08.2003**
(21) Anmeldenummer: 98933581.5
(22) Anmeldetag: 29.05.1998
(51) Int. Cl.: A61F 2/44

(54) **HÖHENVARIIERBARES WIRBELKÖRPERIMPLANTAT**
HEIGHT-ADJUSTABLE VERTEBRAL IMPLANT
IMPLANT POUR CORPS VERTEBRAL, AJUSTABLE EN HAUTEUR

(30) Priorität: 30.01.1998 DE 19803700
(43) Veröffentlichungstag der Anmeldung: 15.11.2000
(73) Patentinhaber: Königsee Implantate und Instrumente zur Ostheosynthese GmbH, 07426 Königsee (DE)
(72) Erfinder: BÖHM, Heinrich, D-99425 Weimar (DE); BUSCH, Thomas, D-07422 Bad Blankenburg (DE); ORSCHLER, Erich, D-95466 Kirchenpingarten (DE)
(74) Vertreter: Kruspig, Volkmar, Dipl.-Ing.
(86) Internationale Anmeldenummer: EP9803245
(87) Internationale Veröffentlichungsnummer: WO99038460

(56) Entgegenhaltungen:
- WO-A-92/01428
- WO-A-94/18913
- DE-A- 4 328 062
- DE-A- 4 409 392
- DE-A- 4 423 257
- DE-A- 19 509 317
- DE-A- 19 519 101
- DE-A- 19 622 827
- DE-C- 19 500 170
- DE-U- 29 616 778
- FR-A- 2 730 158
- US-A- 5 236 460
- US-A- 5 290 312
- US-A- 5 702 455

## Beschreibung

Die Erfindung betrifft ein höhenvariierbares Wirbelkörperimplantat mit einem ersten im wesentlichen U-förmigen, Ausnehmungen oder Durchbrüche aufweisenden Korb sowie am Korb ausgebildeten Wirbelstützflächen gemäß Oberbegriff des Patentanspruches 1.

Aus der DE-U-29616778 ist ein Wirbelkörperimplantat mit zwei teleskopartig ineiander geführten Körben bekannt, die an ihren Stirnseiten zackenartige Arretierungsvorsprünge aufweisen, und die in einer vorgegebenen Endlage gegeneinander fixierbar sind.

Aus der DE 43 28 062 A1 ist ein Stützstab mit seitlich aufschiebbaren Implantatkörpern bekannt. Um ein Aufschieben zu ermöglichen, besitzen die Implantatkörper eine seitliche Nut, wobei eine Oberflächenstruktierung in Form einer Verzahnung der Fixierung der Flächen des Implantatkörpers untereinander sowie darüber hinaus dem Lageerhalt bezüglich des bzw. der Wirbelkörper dient.
Dadurch, daß die dort gezeigten Implantatkörper in unterschiedlicher Höhe vorgefertigt und auf den Stützstab aufschiebbar sind, kann in gewisser Weise eine Höhenanpassung erfolgen. Die Handhabung des Implantats ist jedoch unter operativen Bedingungen außerordentlich erschwert und die Herstellungskosten sind hoch.

Aus der EP 0 302 719 ist ein Implantat in U-Form bekannt, wobei dort Öffnungen vorgesehen sind, die der Aufnahme von Knochenzement dienen. Weitere Öffnungen können als Aufnahme für eine Schraube vorhanden sein, um das Implantat unmittelbar im Wirbel zu fixieren. Als Material wird für das gezeigte Implantat auf kohlefaserverstärkten Kunststoff verwiesen.

Der Implantat-Platzhalter nach EP 0 268 115 A1 besitzt ein Zylindermantelelement mit einer Vielzahl von rautennetzförmigen Ausnehmungen. Ein dort vorgesehener Ring am oberen und unteren Ende des Zylindermantelelements verhindet ein unerwünschtes zu tiefes Eindringen des Implantats in den Wirbel. Dem gleichen Zweck dient eine zusätzlich vorgesehene Bodenplatte. Das Zylindermantelelement ist geschlossen und besitzt neben den Rautenöffnungen keine weiteren zum Einbringen von Knochenzement, so daß auch hier Schwierigkeiten bei der operativen Handhabung bestehen mit Nachteilen beim Knochenzementeinpressen.

Die DE 196 15 938 A1 offenbart eine Wirbelsäulen-Stützvorrichtung zur Durchführung einer intersomatischen Arthrodese. Die Stützvorrichtung besteht aus Titan und ist flach und langgestreckt ausgebildet und in ihrem Mittelbereich verbreitert. Die Kanten besitzen mit Ausnahme des Mittelbereichs ein Sägezahnprofil. Die Gestalt der vorgestellten Stützvorrichtung ist der eines offenen Ringes ähnlich, wobei durch das Aufbiegen der Endbereiche die Stützvorrichtung durchmesserseitig verschiedenen Anwendungsfällen angepaßt werden kann.

Ein höhenvariierbarer Wirbelkörperersatz mit einer Hülse und einem in axialer Richtung verschiebbaren ersten Widerlagerkörper bzw. einem zweiten Widerlager ist aus der DE 44 09 392 A1 bekannt. Zum Bewegen der Widerlagerkörper untereinander ist eine Gewindevorrichtung vorgesehen. Ein ähnliches Prinzip ist in der DE 44 23 257 A1 gezeigt.

Eine Gewindeverstellbarkeit wird auch in der DE 195 19 101 A1 erwähnt, wobei dort an den Enden der Hülsenteile des Wirbelkörperersatzes zackenförmige Ausnehmungen vorgesehen sind, um ein radiales Arretieren gegenüber den Wirbeln zu realisieren. Die zylindrischen Teile des Wirbelkörperersatzes besitzen eine Vielzahl von Ausnehmungen, um das Durchwachsen mit Körpermaterial zu verbessern. Das Einsetzen des Wirbelkörperersatzes aus DE 195 19 101 A1 ist jedoch außerordentlich problematisch, da hierfür ein entsprechendes Aufspreizen der Wirbel erfolgen muß. Darüber hinaus besitzt das bekannte Implantat eine zu große metallische Masse mit entsprechenden nachteiligen postoperativen Folgen.

Die DE 195 09 317 A1 zeigt ein Implantat zum Einsetzen zwischen Wirbelkörper der Wirbelsäule als Platzhalter, wobei zwei endständige Implantatteile vorgesehen sind, die mit einem dazwischen befindlichen mittigen Implantatteil, das mit den endständigen Implantatteilen drehbar verbunden ist, wechselwirkt. Die gezeigten Implantatteile sind als rohrförmige Hülse bzw. als Stirnring ausgebildet. Durch vorgesehene Öffnungen kann Knochenzement eingebracht werden. Diese Öffnungen sind jedoch nur dann optimal zum Einbringen von Knochenzement geeignet, wenn diese zur Deckung gebracht werden, was jedoch bei dem gewählten Verstellprinzip problematisch ist.

Hinsichtlich der Verstellbarkeit sei noch auf die US 5,236,460 verwiesen, die eine Teleskopanordnung eines Implantats zeigt, wobei mit Hilfe eines speziellen Werkzeuges ein aushärtendes Material in den Teleskopraum verbracht werden kann. Die EP 0 637 439 A1 hingegen zeigt ein Implantat mit einem Keilgetriebe zur Höhenverstellung. Zusätzlich können dort Arretierungszacken herausgeschwenkt werden, um das Implantat nach dem Einbringen zwischen den Wirbeln zu verkrallen.

Ausgehend vom Vorgenannten ist es daher Aufgabe der Erfindung, ein weiterentwickeltes höhenvariierbares Wirbelkörperimplantat anzugeben, das bei geringstem Materialaufwand eine ausgezeichnete Stabilität aufweist, welches operativ besonders einfach zu handhaben ist und das optimierte Öffnungen zum Einbringen von Knochenzement bei gleichzeitig gegebener Höhenvariierbarkeit umfaßt.

Die Lösung der Aufgabe der Erfindung erfolgt mit einem Gegenstand gemäß Definition nach Patentanspruch 1, wobei die Unteransprüche mindestens zweckmäßige Ausgestaltungen und Weiterbildungen umfassen.

Der Grundgedanke der Erfindung besteht nun darin, einen inneren, längsseitig offenen, im wesentlichen U-förmigen Korb von einem äußeren, im wesentlichen gleich gestalteten Korb zu umgreifen, wobei beide Körbe gegeneinander teleskopartig geführt sind. Zusätzlich sind Arretierungen in mindestens zwei Endpositionen möglich. Die Höhenverstellung erfolgt entweder mit einem von der offenen Seite der Körbe einzuführenden speziellen Werkzeug oder durch Nutzung einer vorgesehenen Verzahnung im Zusammenwirken mit einer Verstellschraube.

Es sei angemerkt, daß unter U-förmig auch eine mehreckige Form, eine halbkreis- oder kreisförmige Zylinderform oder dergleichen zu verstehen ist, d.h. wesentlich soll bei den erfindungsgemäßen Körben die Möglichkeit des teleskopartigen Ineinanderführens und der gegenseitigen Abstützung der Seitenwände sein, wobei gewährleistet ist, daß sich eine durchgehende seitliche Öffnung einstellt.

Neben der Endlagefixierung durch vorzugsweise Schrauben sind metallische Zungen vorgesehen, die mit entsprechenden Ausnehmungen verkrallbar sind, so daß sich die Stabilität der Anordnung trotz der unter dem Aspekt des Einbringens von Knochenzement offenen U-Form erhöht. Abgewinkelte zungenartige Wirbelstützflächen sowie in Längsrichtung sich erstreckende zackenartige Arretierungsvorsprünge verhindern ein Verdrehen des Wirbelkörperimplantats nach dem Einsetzen und ein unerwünschtes zu tiefes Eindringen des Implantats in die benachbarten Wirbel postoperativ.

Anwendungsseitig wird zunächst der innere Korb in den äußeren Korb hineingefahren, d.h. das Implantat wird mit derart eingeschobenem Innenteil zwischen den Wirbeln positioniert, wobei anschließend das Herausbewegen des Innenteils bis zur Endposition bzw. das Ausführen einer Relativbewegung der Körbe zueinander erfolgt. In der Endposition kann mit einer entsprechenden Schraube das Befestigen des Innenteils bezogen auf das Außenteil, d.h. der Körbe untereinander vorgenommen werden, wobei stabiltitätserhöhend ein Verklinken oder Verkrallen durch die vorzugsweise im Außenteil vorhandene Metallzunge in Verbindung mit der entsprechenden Ausnehmung im Innenteil möglich ist.

In der Ausführungsform mit Langloch im inneren Korb und dort vorgesehener Verzahnung besteht die Möglichkeit der kontinuierlichen Höhenverstellung durch Einführen eines Werkzeuges über die offene Seite der U-förmigen Körbe.

Beim Erfindungsgegenstand wird, wie oben dargestellt, also von einem ersten, inneren Korb ausgegangen, der von einem zweiten, äußeren U-förmigen Korb umgriffen und geführt ist. Die Schenkel des inneren und äußeren Korbes sind so ausgerichtet, daß sich eine durchgehende seitliche Öffnung ergibt. Diese durchgehende seitliche Öffnung zeigt bei der Anwendung zum Operateur und ermöglicht sowohl das Einführen des Werkzeugs zur dargelegten Höhenverstellung resp. zur Arretierung als auch das optimale Einbringen von Knochenzement.

Jeweils eine der Stirnseiten der Körbe weist obere und untere abgewinkelte, zungenartige Wirbelstützflächen sowie sich in Längsrichtung erstreckende zackenartige Arretierungsvorsprünge auf. Diese Wirbelstützflächen bzw. Arretierungsvorsprünge können bei der Ausführungsform als metallisches Implantat leicht durch Stanzen und Biegen erzeugt werden, so daß die Herstellungskosten gering gehalten werden.

Um die Stabilität des Wirbelkörperimplantats zu erhöhen, besitzen die Schenkel des inneren und äußeren Korbes an ihren Schenkelenden jeweils einen nach innen gerichteten Krümmungsabschnitt. Hierdurch wird trotz der erwähnten durchgehenden seitlichen Öffnung bei einwirkenden Druck- und/oder Rotationskräften die gewünschte vorgegebene Lagezuordnung erhalten, ohne daß weitere stabilisierende Mittel notwendig werden.

Vorzugsweise im Bereich dieser Krümmungsabschnitte sind zum Fixieren der Körbe in ihrer Endlage jeweils Aussparungen einerseits und Zungen andererseits vorgesehen, wobei Zunge und Aussparung miteinander verklink- oder verkrallbar sind.

Ergänzend besteht die Möglichkeit, daß mindestens im Bereich der Krümmungsabschnitte der sich gegenüberliegenden Flächen der Körbe eine vorzugsweise geprägte Rastverzahnung vorhanden ist, um ein Verrutschen oder unerwünschtes Verdrehen der Körbe gegeneinander auszuschließen.

Im Bereich des die Schenkel verbindenden Teiles der U-förmigen Körbe sind Bohrungen zur Aufnahme einer Feststellschraube angeordnet, wobei die entsprechende Bohrung des äußeren Korbes ein Gewinde umfaßt.

Die Höhenvariierbarkeit des Wirbelkörperimplantats wird in einer bevorzugten Ausführungsform dadurch realisiert, daß der innere Korb vorzugsweise im Bereich des die Schenkel verbindenden Teiles ein sich in Längsrichtung erstreckendes erstes Langloch mit einer Verzahnung aufweist, um hier im Zusammenwirken mit einer Stellschraube im äußeren Korb eine Relativbewegung und entsprechende Verstellung zwischen den Körben zu erreichen.
Ein weiteres, zweites Langloch ist vorzugsweise dem ersten Langloch benachbart vorgesehen und dient dem Feststellen der Einstellposition mittels Feststellschraube.

Um das Eindringen der Arretierungsvorsprünge bis zum Inanschlagkommen der zungenartigen Wirbelstützflächen bezogen auf die angrenzenden Wirbel zu erleichtern, ohne starke Knochenschädigungen hervorzurufen, besitzen die Arretierungsvorsprünge, die beispielsweise Dreiecksform aufweisen, einen Schneidschliff.

Die Arretierungsvorsprünge und Wirbelstützflächen, die jeweils an einer der Stirnseiten des ersten und des zweiten Korbes vorgesehen sind, werden zweckmäßigerweise alternierend angeordnet, um die gewünschte Planlage des Implantats sicherzustellen.

Die Erfindung soll nachstehend anhand eines Ausführungsbeispiels sowie unter Zuhilfenahme von Figuren näher erläutert werden.

Hierbei zeigen:
- Fig. 1a: eine Seitenansicht des inneren Korbes des Wirbelkörperimplantats;
- Fig. 1b: eine Seitenansicht des inneren Korbes des Wirbelkörperimplantats mit erkennbarem Öffnungsbereich;
- Fig. 1c: eine Draufsicht des inneren Korbes des Wirbelkörperimplantats:
- Fig. 1d: einen Schnitt längs der Linie A-A nach Fig. 1b;
- Fig. 2: eine Darstellung des Zuschnitts für einen inneren Korb des Wirbelkörperimplantats mit vorgesehener Langlochverzahnung zur kontinuierlichen Höhenvariierbarkeit und
- Fig. 3: eine perspektivische Darstellung des kompletten Wirbelkörperimplantats umfassend inneren ersten und äußeren zweiten U-förmigen Korb.

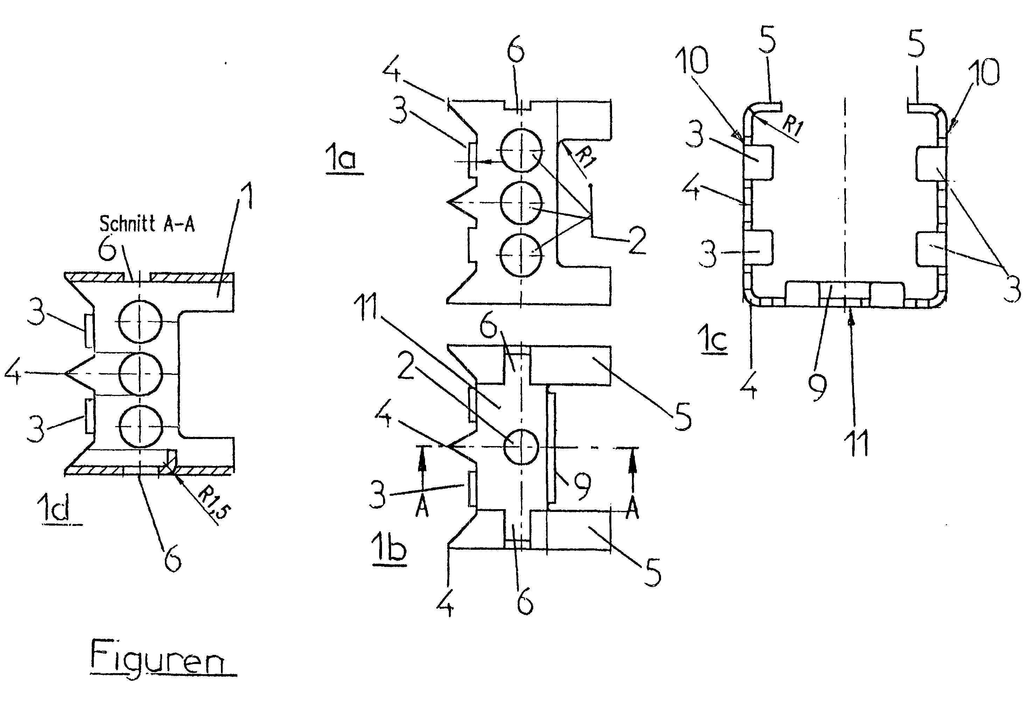

Wie aus der Fig. 1a erkennbar, umfaßt der innere erste Korb 1 in seinen Schenkeln vorgesehene Ausnehmungen oder Durchbrüche 2.
An einer Stirnseite des ersten inneren Korbes 1 sind abgewinkelte zungenartige Wirbelstützflächen 3 sowie abwechselnd zu diesen Stützflächen 3 sich in Längsrichtung erstreckende zackenartige Arretierungsvorsprünge 4 vorgesehen. An ihrer Spitze können die Arretierungsvorsprünge einen Schliff 16 zum leichteren Eindringen in die jeweils gegenüberliegende Fläche des Wirbels aufweisen.

In einem nach innen gerichteten Krümmungsabschnitt 5 (siehe auch Fig. 1c) ist eine Aussparung 6 vorgesehen, die mit einer Zunge 7 des äußeren Korbes 8 durch Verbiegen verklink- oder verkrallbar ist.

Wie aus den Fig. 1a bis 1d ersichtlich, wurde die Fläche und die Form so optimiert, daß einerseits eine geringe Metallmenge gegeben ist, andererseits aber die mechanische Stabilität den insbesondere postoperativen Anforderungen genügt. Stabilitätserhöhend wirkt beispielsweise auch die in den Fig. 1b und 1c erkennbare Anschlagkante 9.

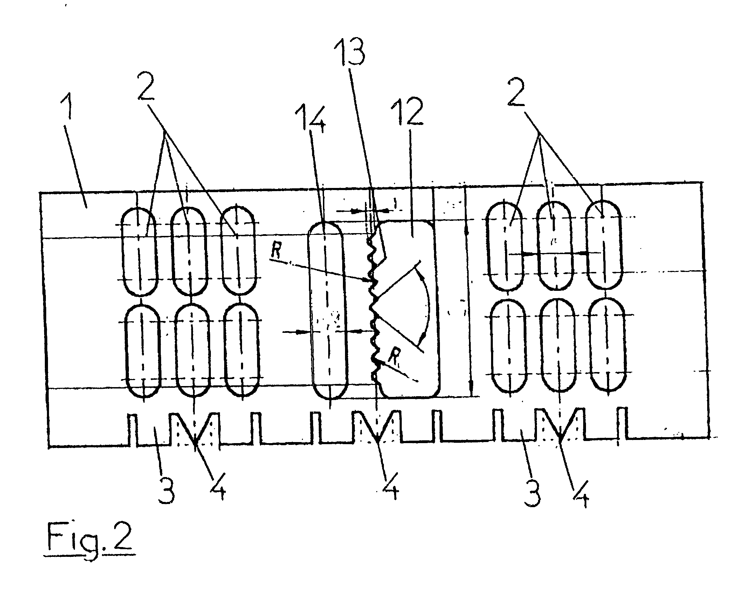

Fig. 2 stellt einen Zuschnitt des ersten inneren Korbes 1 dar, wobei auch hier bereits die vorbereiteten Flächen für die Wirbelabstützung 3 als auch die Arretierungsvorsprünge 4 erkennbar sind.
Beim Ausführungsbeispiel nach Fig. 2 ist im Bereich des die Schenkel 10 verbindenden Teiles 11 ein sich in Längsrichtung erstreckendes erstes Langloch 12 mit einer Verzahnung 13 ausgebildet. Ein zweites Langloch 14 ist dem ersten Langloch 12 im wesentlich parallel verlaufend benachbart angeordnet und dient dem Feststellen der gewählten Einstell- oder Verstellposition mittels einer Feststellschraube, die sich in einer Gewindebohrung des zweiten äußeren Korbes 8 befindet.

Angemerkt werden soll, daß, obwohl zeichnerisch nicht dargestellt, im Bereich der Krümmungsabschnitte an oder auf den sich gegenüberliegenden Oberflächen des ersten inneren Korbes 1 und und des äußeren zweiten Korbes 8 eine Verzahnung vorgesehen sein kann, die ein unerwünschtes Verschieben oder Verdrehen der Körbe gegeneinander verhindert.

Wie insbesondere aus der Darstellung nach Fig. 2 ersichtlich, können die Körbe aus einem metallischen Band gestanzt und anschließend durch Biegen geformt werden, so daß die Fertigungskosten insgesamt gering gehalten werden. Als metallisches Material wird bei einem Ausführungsbeispiel Titanblech in einer Dicke von im wesentlichen 1 bis 1,5 mm verwendet.

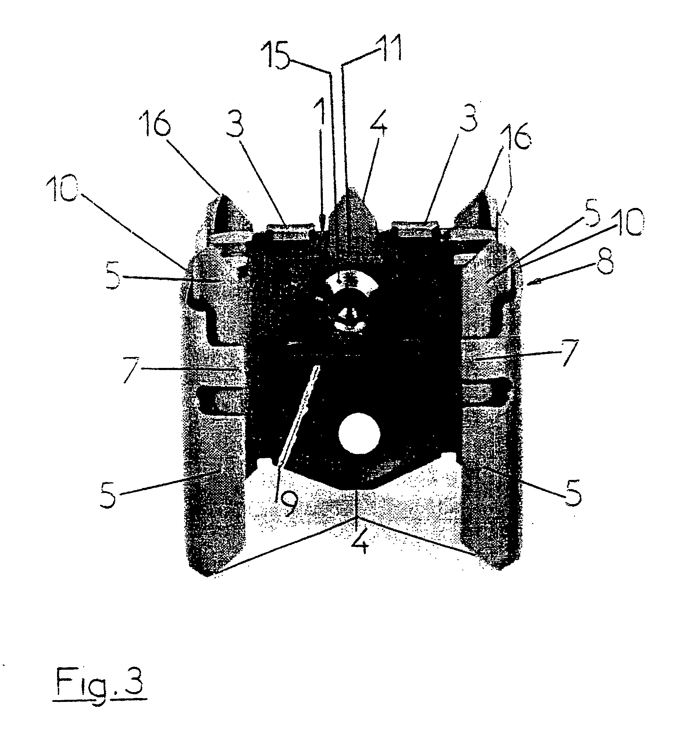

Die Fig. 3 stellt ein einsatzbereites Wirbelkörperimplantat dar.
Wie ersichtlich, ist der erste innere Korb 1 vom zweiten äußeren Korb 8 umfaßt. Eine Feststellschraube 15 ist eingebracht und kann von der sichtbaren offenen Seite leicht betätigt werden. Die Krümmungsabschnitte 5 des äußeren Korbes 8 lassen die Zungen 7, hier noch im nichtverkrallten Zustand, erkennen.
Die Verkrallung ist mittels eines zangenartigen Werkzeuges, welches in den offenen Bereich eingeführt wird, leicht möglich, ohne daß unerwünschte Querkräfte auf das Implantat gelangen und dies möglicherweise in seiner vorfixierten Lage verschieben. Ebenfalls ist anhand der Darstellung nach Fig. 3 die Ausbildung des Schneidschliffs 16 an den zackenartigen Arretierungsvorsprüngen 4 erkennbar.

Das Wirbelkörperimplantat wird mit der geschlossenen Seite der U-förmigen Körbe voran eingesetzt, wobei nach dem Einsetzen zwischen leicht aufgespreizte, benachbarte Wirbel der innere Korb 1 ausgefahren und arretiert wird. Durch die Schenkelöffnung besteht sowohl die Möglichkeit, die entsprechenden Arretierungswerkzeuge einzuführen und zu handhaben als auch in optimaler Weise ein Verfüllen mit Knochenzement oder dergleichen vorzunehmen.

Alles in allem gelingt es mit dem Wirbelkörperimplantat der gezeigten Art, die Wirbelsäule sicher zu stützen, ohne daß die Menge des zur Stützung erforderlichen Fremdkörpermaterials eine kritische Schwelle übersteigt. Die Handhabung des Implantats selbst ist unkompliziert und durch die Höhenvariierbarkeit bezogen auf den Stand der Technik wesentlich erleichtert. Durch die Ausbildung der ineinander geführten Körbe des Implantats aus einem metallischen, vorzugsweise Titanblech mittels Stanzen und Biegen können die Fertigungskosten reduziert werden.

### Bezugszeichenaufstellung

- 1: innerer Korb
- 2: Ausnehmungen, Durchbrüche
- 3: Wirbelstützfläche
- 4: Arretierungsvorsprünge
- 5: Krümmungsabschnitt
- 6: Aussparung
- 7: Zunge
- 8: äußerer Korb
- 9: Anschlagkante
- 10: Schenkel
- 11: Verbindungsteil der Schenkel
- 12: erstes Langloch
- 13: Verzahnung
- 14: zweites Langloch
- 15: Feststellschraube
- 16: Schneidschliff

## Patentansprüche

1. Höhenvariierbares Wirbelkörperimplantat mit einem ersten, im wesentlichen U-förmigen, Ausnehmungen oder Durchbrüche aufweisenden Korb sowie am Korb ausgebildeten Wirbelstützflächen,
**dadurch gekennzeichnet, daß**
der erste Korb ein innerer Korb (1) ist, welcher von einem zweiten, äußeren U-förmigen Korb (8) umgriffen und teleskopartig geführt ist, wobei die Schenkel (10) des inneren und äußeren Korbes (1; 8) so ausgerichtet sind, daß sich eine durchgehende seitliche Öffnung ergibt,
jeweils eine der Stirnseiten der Körbe (1; 8) obere und untere abgewinkelte, zungenartige Wirbelstützflächen (3) sowie sich in Längsrichtung erstreckende, zackenartige Arretierungsvorsprünge (4) aufweisen, und
daß der innere und äußere Korb (1; 8) in einer vorgegebenen Endlage gegeneinander fixierbar sind.

2. Höhenvariierbares Wirbelkörperimplantat nach Anspruch 1,
**dadurch gekennzeichnet, daß**
die Schenkel (10) des inneren und äußeren Korbes (1; 8) an ihren Schenkelenden jeweils einen nach innen gerichteten Krümmungsabschnitt (5) aufweisen.

3. Höhenvariierbares Wirbelkörperimplantat nach Anspruch 2,
**dadurch gekennzeichnet, daß**
im Bereich der Krümmungsabschnitte (5) zum Fixieren der Körbe (1; 8) in ihrer Endlage jeweils mindestens eine Aussparung (6) einerseits und mindestens eine Zunge (7) andererseits vorgesehen und Zunge (7) sowie Aussparung (6) miteinander verklinkoder verkrallbar sind.

4. Höhenvariierbares Wirbelkörperimplantat nach Anspruch 2,
**dadurch gekennzeichnet, daß**
mindestens im Bereich der Krummungsabschnitte (5) die sich gegenüberliegenden Flächen des inneren und äußeren Korbes (1; 8) eine Rastverzahnung aufweisen.

5. Höhenvariierbares Wirbelkörperimplantat nach einem der vorangegangenen Ansprüche,
**dadurch gekennzeichnet, daß**
vorzugsweise im Bereich (11) des die Schenkel (5) verbindenden Teiles der U-förmigen Körbe (1; 8) Bohrungen zur Aufnahme einer Feststellschraube (15) angeordnet sind, wobei die entsprechende mindestens eine Bohrung im äußeren Korb (8) ein Gewinde aufweist.

6. Höhenvariierbares Wirbelkörperimplantat nach einem der Ansprüche 1 bis 4,
**dadurch gekennzeichnet, daß**
der innere Korb (1) vorzugsweise im Bereich (11) des die Schenkel (10) verbindenden Teiles ein sich in Längsrichtung erstreckendes erstes Langloch (12) mit einer Verzahnung (13) aufweist, um im Zusammenwirken mit einer Stellschraube im äußeren Korb (8) eine Relativbewegung und Verstellung zwischen den Körben (1; 8) zu ermöglichen.

7. Höhenvariierbares Wirbelkörperimplantat nach Anspruch 6,
**dadurch gekennzeichnet, daß**
zum Feststellen der Einstellposition mittels Feststellschraube ein weiteres, zweites Langloch (14) dem ersten Langloch (12) benachbart vorgesehen ist.

8. Höhenvariierbares Wirbelkörperimplantat nach einem der vorangegangenen Ansprüche,
**dadurch gekennzeichnet, daß**
die zackenartigen Arretierungsvorsprünge (4) zum leichteren Eindringen in die Wirbelkörperflächen einen Schneidschliff (16) aufweisen.

9. Höhenvariierbares Wirbelkörperimplantat nach einem der vorangegangenen Ansprüche.
**dadurch gekennzeichnet, daß**
die jeweils an einer der Stirnseiten des ersten und zweiten Korbes (1; 8) vorgesehenen Wirbelstützflächen (3) und Arretierungsvorsprünge (4) alternierend angeordnet sind.

10. Höhenvariierbares Wirbelkörperimplantat nach einem der vorangegangenen Ansprüche,
**dadurch gekennzeichnet, daß**
die Körbe aus einem metallischen Stanz- und Hiegeteil, vorzugsweise Titan bestehen.

11. Höhenvariierbares Wirbelkörperimplantat nach einem der vorangegangenen Ansprüche
**dadurch gekennzeichnet, daß**
der erste und der zweite Korb (1; 8) einen mehreckigen, kreisförmigen oder halbkreisförmigen Querschnitt aufweisen.

## Claims

1. A height-adjustable vertebral implant, comprising a first essentially U-shaped basket with recesses or break-outs and vertebra supporting faces formed at the basket,
**characterised in that**
the first basket is an inner basket (1) which is encompassed by a second outer U-shaped basket (8) and telescopically guided, with the legs (10) of the inner and outer basket (1; 8) being oriented in such a manner that a continuous lateral opening is created,
one each of the faces of the baskets (1; 8) comprises upper and lower angled tongue-type vertebra supporting faces (3) as well as prong-type locking projections (4) extending in the longitudinal direction, and
that the inner and outer basket (1; 8) can be secured relative to one another in a predetermined end position.

2. The height-adjustable vertebral implant according to Claim 1,
**characterised in that**
the legs (10) of the inner and outer basket (1; 8) comprise one inwardly directed bend portion (5) each at their leg ends.

3. The height-adjustable vertebral implant according to Claim 2,
**characterised in that**
at least one recess (6) on the one hand and at least one tongue (7) on the other hand are provided in the area of the bend portion (5) for securing the baskets (1; 8) in their end position, and the tongue (7) as well as the recess (6) can be latched or clutched with one another.

4. The height-adjustable vertebral implant according to Claim 2,
**characterised in that**
the opposite faces of the inner and outer basket (1; 8) comprise a detent toothing at least in the area of the bend portions (5).

5. The height-adjustable vertebral implant according to one of the previous claims,
**characterised in that**
holes for accommodating a set screw (15) are arranged preferably in the area (11) of the portion of the U-shaped baskets (1; 8) joining the legs (10), with the respective at least one hole in the outer basket (8) comprising a thread.

6. The height-adjustable vertebral implant according to one of Claims 1 to 4,
**characterised in that**
the inner basket (1), preferably in the area (11) of the portion joining the legs (10), comprises a first elongated hole (12) extending in the longitudinal direction with a toothing (13), in order to enable a relative movement and adjustment between the baskets (1; 8) in cooperation with a set screw in the outer basket (8).

7. The height-adjustable vertebral implant according to Claim 6,
**characterised in that**
a further, second elongated hole (14) is provided neighbouring the first elongated hole (12) for securing the adjusted position my means of a set screw.

8. The height-adjustable vertebral implant according to one of the previous claims,
**characterised in that**
the prong-type locking projections (4) comprise a cutting edge (16) for facilitating the penetration into the faces of the vertebra body.

9. The height-adjustable vertebral implant according to one of the previous claims,
**characterised in that**
the vertebra supporting faces (3) and locking projections (4) respectively provided at one of the faces of the first and second basket (1; 8) are arranged alternatingly.

10. The height-adjustable vertebral implant according to one of the previous claims,
**characterised in that**
the baskets consist of a metallic punching or bent part, preferably in titanium.

11. The height-adjustable vertebral implant according to one of the previous claims,
**characterised in that**
the first and the second basket (1; 8) comprise a polygonal, circular, or semi-circular cross-section.

## Revendications

1. Implant pour corps vertébral, ajustable en hauteur, comportant un premier panier sensiblement en forme de U et présentant des évidements ou des traversées ainsi que des surfaces d'appui de vertèbre réalisées sur le panier, **caractérisé en ce que**
le premier panier est un panier intérieur (1) qui est entouré par un deuxième panier extérieur (8) en forme de U et qui est guidé de façon télescopique, les branches (10) des paniers intérieur et extérieur (1 ; 8) étant orientées de telle sorte qu'il résulte une ouverture latérale traversante,
l'une des faces frontales respectives des paniers (1 ; 8) comprend des surfaces d'appui de vertèbre (3) supérieure et inférieure, coudées et en forme de languette ainsi que des saillies d'arrêt (4) en forme de dent s'étendant en direction longitudinale, et
les paniers intérieur et extérieur (1 ; 8) sont susceptibles d'être fixés l'un par rapport à l'autre dans une position finale prédéterminée.

2. Implant pour corps vertébral, ajustable en hauteur selon la revendication 1, **caractérisé en ce que** les branches (10) des paniers intérieur et extérieur (1 ; 8) présentent à leurs extrémités un tronçon courbé respectif (5) dirigé vers l'intérieur.

3. Implant pour corps vertébral, ajustable en hauteur selon la revendication 2, **caractérisé en ce qu'**il est prévu dans la zone des tronçons courbés (5), afin de fixer les paniers (1 ; 8) dans leur position finale, au moins une échancrure respective (6) d'une part et au moins une languette respective (7) d'autre part, et **en ce que** la languette (7) et l'échancrure (6) sont susceptibles d'être encliquetées ou enclenchées l'une dans l'autre.

4. Implant pour corps vertébral, ajustable en hauteur selon la revendication 2, **caractérisé en ce qu'**au moins dans la zone des tronçons courbés (5), les surfaces opposées des paniers intérieur et extérieur (1 ; 8) présentent une denture d'enclenchement.

5. Implant pour corps vertébral, ajustable en hauteur selon l'une des revendications précédentes, **caractérisé en ce qu'**il est prévu, de préférence dans la zone (11) de la partie des paniers en forme de U (1 ; 8) reliant les branches (5), des percages pour recevoir une vis d'immobilisation (15), ledit au moins un perçage correspondant présentant un taraudage dans le panier extérieur (8).

6. Implant pour corps vertébral, ajustable en hauteur selon l'une des revendications 1 à 4, **caractérisé en ce que** le panier intérieur (1) présente, de préférence dans la zone (11) de la partie reliant les branches (10), un premier trou oblong (12) s'étendant en direction longitudinale et pourvu d'une denture (13) pour permettre un mouvement relatif et une immobilisation entre les paniers (1 ; 8), en coopération avec une vis de positionnement dans le panier extérieur (8).

7. Implant pour corps vertébral, ajustable en hauteur selon la revendication 6, **caractérisé en ce que** pour immobiliser la position de réglage au moyen de la vis d'immobilisation, il est prévu un autre deuxième trou oblong (14) au voisinage du premier trou oblong (12).

8. Implant pour corps vertébral, ajustable en hauteur selon l'une des revendications précédentes, **caractérisé en ce que** les saillies d'arrêt (4) en forme de dent présentent un tranchant meulé (16) pour faciliter la pénétration dans les surfaces de corps vertébral.

9. Implant pour corps vertébral, ajustable en hauteur selon l'une des revendications précédentes, **caractérisé en ce que** les surfaces d'appui de vertèbre (3) prévues sur l'une des faces frontales respectives du premier et du deuxième panier (1 ; 8) et les saillies d'arrêt (4) sont agencées en alternance.

10. Implant pour corps vertébral, ajustable en hauteur selon l'une des revendications précédentes, **caractérisé en ce que** les paniers sont constitués par une pièce métallique poinçonné et cintrée, de préférence en titane.

11. Implant pour corps vertébral, ajustable en hauteur selon l'une des revendications précédentes, **caractérisé en ce que** le premier et le deuxième panier (1 ; 8) présentent une section transversale polygonale, circulaire ou demi-circulaire.
